# EUROPÄISCHE PATENTSCHRIFT

(11) **EP 0 692 229 B1**
(45) Veröffentlichungstag und Bekanntmachung des Hinweises auf die Patenterteilung: **22.12.1999**
(21) Anmeldenummer: 95110634.3
(22) Anmeldetag: 07.07.1995
(51) Int. Cl.: A61F 2/46

(54) **Vorrichtung zum Mischen und Austragen von Knochenzement**
Mixing and dispensing device for bone cement
Dispositif pour mélanger et appliquer un ciment osseux

(30) Priorität: 16.07.1994 DE 4425218
(43) Veröffentlichungstag der Anmeldung: 17.01.1996
(73) Patentinhaber: MERCK PATENT GmbH, 64293 Darmstadt (DE)
(72) Erfinder: Hauke, Günter, D-64367 Mühltal (DE); Funk, Reiner, D-64839 Münster-Altheim (DE); Specht, Rainer, Dr., D-90765 Fürth (DE); Martin, Peter, D-65205 Wiesbaden (DE); Krotz, Ralf, Dr., D-69168 Wiesloch (DE); Nies, Berthold, Dr., D-64407 Fränkisch-Crumbach (DE)

(56) Entgegenhaltungen:
- EP-A- 0 397 589
- DE-A- 3 208 472
- DE-A- 3 640 279
- DE-A- 4 302 230

## Beschreibung

Die Erfindung betrifft eine Vorrichtung zum Mischen und Austragen von Knochenzement mit einem zweiteiligen Mischzylinder, in dem ein durchbrochener Mischkolben mittels einer an einem Zylinderende abgedichtet herausgeführten Betätigungsstange axial und drehbar bewegbar ist, wobei der Mischzylinder am anderen Zylinderende durch einen lösbar verriegelten Austragskolben verschlossen ist, der axial in den Mischzylinder eindrückbar ist, um den gemischten Knochenzement durch eine gegenüberliegende Austragsöffnung auszutragen.

Die Ausgangsmaterialien zur Erzeugung von Knochenzement, nämlich ein pulverförmiges Polymer und ein flüssiges Monomer, müssen unmittelbar vor dem Einbringen des Knochenzements in den Knochen gemischt werden. Dieser Mischvorgang muß daher im Operationssaal unter sterilen Bedingungen erfolgen.

Hierfür sind unterschiedliche Vorrichtungen bekannt geworden, denen gemeinsam ist, daß ein Mischzylinder, in dem das Pulverförmige Polymer und das flüssige Monomer gemischt werden, unmittelbar danach als Austragszylinder bzw. Kartusche dient, aus dem der fertig gemischte Knochenzement herausgepreßt und an die Verwendungsstelle gebracht wird.

Üblicherweise wird beim Mischvorgang oder unmittelbar danach ein Vakuum im Mischzylinder erzeugt, um die Porenbildung im Knochenzement zumindest wesentlich herabzusetzen.

Bei einer bekannten Vorrichtung der eingangs genannten Gattung (DE 43 02 230 A1) müssen die Ausgangsmaterialien, nämlich Pulver und Flüssigkeit, vor dem Mischvorgang in den Mischzylinder eingebracht werden, der erst danach verschlossen wird. Durch Betätigung des Mischkolbens mittels der Betätigungsstange werden die Materialien im Mischzylinder innig gemischt, wobei der Austragskolben am Zylinderende festgehalten wird. Nach Beendigung des Mischvorgangs wird der Austragskolben freigegeben und als Preßkolben in den Mischzylinder eingedrückt. Der gemischte Knochenzement wird am gegenüberliegenden Zylinderende ausgetragen. Beim freien Einfüllen von Flüssigkeit und insbesondere Pulver besteht die Gefahr, daß dieses Material in die Umgebung gelangt und zu Verunreinigungen führt, die insbesondere unter Operationsbedingungen unerwünscht sind. Als besonders störend wird dabei die Möglichkeit empfunden, daß das Pulver beim Einfüllen zu einer Staubentwicklung führen kann und daß das flüssige Monomer Dämpfe abgeben kann. Deshalb ist es erforderlich, das Einfüllen der Ausgangsmaterialien in den Mischzylinder in möglichst großer Entfernung vom Operationsfeld durchzuführen.

Aufgabe der Erfindung ist es, eine Vorrichtung der eingangs genannten Gattung so auszubilden, daß einerseits das pulverförmige Polymer und andererseits das flüssige Monomer so vorkonfektioniert werden können, daß diese Materialien in abgeschlossenen Behältnissen vorbereitet und in den Operationsbereich gebracht werden können, ohne daß sie vor dem Mischvorgang noch einmal umgefüllt werden müssen.

Diese Aufgabe wird erfindungsgemäß dadurch gelöst, daß zwischen dem Mischkolben und dem Austragskolben ein Trennkolben axial verschiebbar im Mischzylinder angeordnet ist und daß die durch den Trennkolben voneinander getrennten Zylinderräume des Mischzylinders mindestens in einem dem Austragskolben benachbarten Bereich durch mindestens einen Überströmungskanal miteinander verbunden sind.

Zur Vorbereitung für den Mischvorgang wird das pulverförmige Polymer in den auch die Austragsöffnung aufweisenden Zylinderteil, in dem sich der Mischkolben befindet, eingefüllt. Sodann wird dieser Zylinderteil mit dem Trennkolben verschlossen.

In den anderen, am Boden mit dem Austragskolben verschlossenen Zylinderteil wird das flüssige Monomer eingebracht, vorzugsweise in einer verschlossenen, leicht zerstörbaren Packung oder Kartusche, beispielsweise einem Folienbeutel.

In dieser vorbereiteten Form werden die Ausgangsmaterialien im Operationsraum bereitgestellt. Vor dem Mischvorgang werden die beiden Zylinderteile miteinander verbunden, um den Mischzylinder zu bilden. Durch Eindrücken der Betätigungsstange werden der Mischkolben und der Trennkolben bei geschlossenem Mischzylinder in Richtung auf den Austragskolben verschoben. Dabei strömt das flüssige Monomer - ggf. nach Zerstörung der die Flüssigkeit enthaltenden Packung - durch einen oder mehrere Überströmungskanäle in den das Pulver enthaltenden, austragsseitigen Zylinderraum. Anschließend erfolgt dort durch Betätigung des Mischkolbens eine intensive Durchmischung von Pulver und Flüssigkeit. Für den anschließenden Austragsvorgang wird der Austragskolben zusammen mit dem darüber befindlichen Trennkolben zur Austragsseite hin verschoben.

Die Handhabung der Vorrichtung ist gegenüber bekannten Ausführungsformen wesentlich vereinfacht, weil die beiden Materialkomponenten in vorkonfektionierter Form bereitgestellt werden und im Operationsraum kein Umfüllen von Flüssigkeit oder Pulver erforderlich ist. Dadurch wird eine Verunreinigung im Operationsbereich durch Staubentwicklung, Dämpfe und/oder Flüssigkeitsspritzer vermieden. So kann vorgesehen sein, daß in den beiden Zylinderräumen des Mischzylinders vorkonfektionierte Ladungen der Mischungskomponenten aufnehmbar sind.

Vorzugsweise sind eine oder mehrere Bohrungen im Trennkolben vorgesehen, die die Überströmungskanäle bilden. Stattdessen ist es auch möglich, die Überströmungskanäle durch Längsnuten in der Innenwand des Mischzylinders zu bilden.

Gemäß einer bevorzugten Ausführungsform der Erfindung ist vorgesehen, daß die Betätigungsstange lösbar mit dem Mischkolben verbunden ist und daß eine abnehmbar am Mischzylinder angebrachte Führungsbuchse, durch die die Betätigungsstange abgedichtet geführt ist, gegen einen Austragsstutzen austauschbar ist.

Dadurch ist es möglich, nach Beendigung des Mischvorgangs die für den Austragsvorgang störende Betätigungsstange zu entfernen. Die Öffnung, in der die Führungsbuchse für die Betätigungsstange aufgenommen war, steht anschließend zur Aufnahme eines Austragsstutzens zur Verfügung, so daß auf die Ausbildung einer gesonderten Austragsöffnung verzichtet werden kann.

Gemäß einer besonders bevorzugten Ausführungsform der Erfindung ist vorgesehen, daß die lösbare Verbindung zwischen der Betätigungsstange und dem Mischkolben durch eine Sollbruchstelle gebildet wird. Dadurch kann das Lösen der Betätigungsstange in besonders einfacher Weise und ohne die Anordnung besonderer Kupplungselemente geschehen.

Gemäß einer weiteren vorteilhaften Ausführungsform der Erfindung ist vorgesehen, daß zwischen dem Austragskolben und dem Trennkolben ein axial beweglicher Verschlußkolben angeordnet ist. Dieser zusätzliche Verschlußkolben dient dazu, den unteren, das flüssige Monomer aufnehmenden Zylinderteil vor dem Mischvorgang zu verschließen. In diesem Fall ist der eine, das pulverförmige Polymer enthaltende Zylinderteil durch den Trennkolben und der andere, das flüssige Monomer enthaltende Zylinderteil durch den Verschlußkolben verschlossen, bevor diese beiden Zylinderteile vor dem Mischvorgang miteinander verbunden werden. Wenn zu Beginn des Mischvorgangs in der schon beschriebenen Weise der Trennkolben in Richtung auf den Austragskolben verschoben wird, nimmt er auch den Verschlußkolben mit.

Weitere vorteilhafte Ausgestaltungen des Erfindungsgedankens sind Gegenstand weiterer Unteransprüche.

Nachfolgend werden Ausführungsbeispiele der Erfindung näher erläutert, die in der Zeichnung dargestellt sind. Es zeigt:
Fig. 1 in einem Längsschnitt eine Vorrichtung zum Mischen und Austragen von Knochenzement, wobei die beiden Teile des zweiteiligen Mischzylinders getrennt voneinander dargestellt sind,
Fig. 2-5 aufeinanderfolgende Arbeitsstellungen der in Fig. 1 gezeigten Vorrichtung beim Mischen und Austragen von Knochenzement,
Fig. 6 und 7 die beiden Teile einer gegenüber der Fig. 1 abgewandelten Ausführung einer Vorrichtung zum Mischen und Austragen von Knochenzement,
Fig. 8-12 unterschiedliche Ausführungsbeispiele des Trennkolbens in schematischer Darstellung, und zwar jeweils in einem Schnitt a) und zur Hälfte in einer Draufsicht b), und
Fig. 13 in einem vereinfachten Teil-Längsschnitt den Trennkolben in einem Zylinderabschnitt mit Nuten in der Zylinderwand.

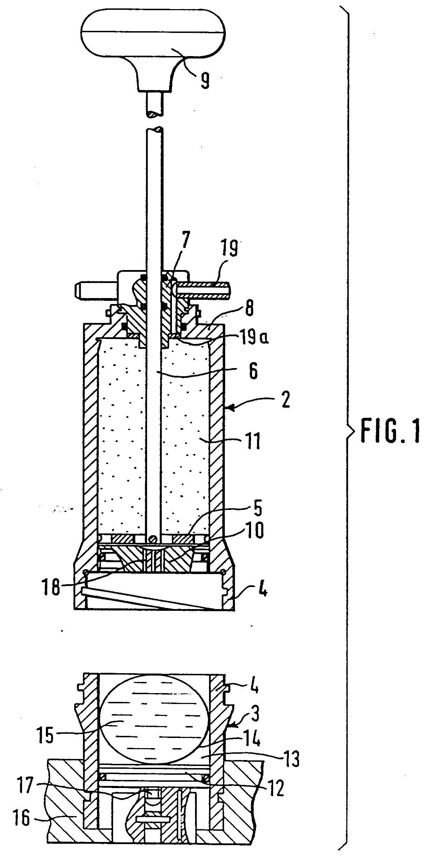

Die in Fig. 1 gezeigte Vorrichtung zum Mischen und Austragen von Kochenzement weist einen zweiteiligen Mischzylinder 1 auf, der aus einem oberen Zylinderteil 2 und einem unteren Zylinderteil 3 besteht. Beide Zylinderteile 2, 3 können mittels einer Schnellkupplung 4 dicht miteinander verbunden werden, beispielsweise mittels einer Verschraubung.

Im oberen Zylinderteil 2 ist ein Mischkolben 5 angeordnet, der beim dargestellten Ausführungsbeispiel aus einer durchbrochenen Platte besteht und der mittels einer Betätigungsstange 6 axial und drehbar bewegbar ist. Die Betätigungsstange 6 ist durch eine Führungsbuchse 7 in der einen Zylinderendwand 8 abgedichtet herausgeführt und trägt an ihrem freien Ende einen Betätigungsgriff 9.

Am unteren Ende des oberen Zylinderteils 2 ist ein Trennkolben 10 angeordnet, der frei beweglich im Mischzylinder 1 aufgenommen ist. In dem in Fig. 1 dargestellten, für einen Misch- und Austragsvorgang vorbereiteten Zustand bildet der Trennkolben 10 den unteren Abschluß des oberen Zylinderteils 2. Der über dem Trennkolben 10 befindliche Zylinderraum 11 enthält ein pulverförmiges Polymer als Ausgangsmaterial für den Knochenzement.

Der untere Zylinderteil 3 ist an seinem unteren Ende durch einen Austragskolben 12 verschlossen und enthält in seinem darüber befindlichen Zylinderraum 13 einen Flüssigkeitsbehälter 14, in dem ein flüssiges Monomer 15 als Ausgangsmaterial für den Knochenzement eingeschlossen ist. Der Flüssigkeitsbehälter 14 kann beispielsweise ein Beutel oder eine Kartusche aus leicht zerstörbarem Material sein, beispielsweise aus Metallfolie.

Bei dem in Fig. 1 gezeigten, für den Mischvorgang vorbereiteten Ausgangszustand ist der untere Zylinderteil 3 in einer Haltevorrichtung 16 aufgenommen, wobei der Austragskolben 12 mittels einer lösbaren Kolbenkupplung 17 arretiert ist.

Der Trennkolben 10 weist durch den Kolben hindurchgehende Bohrungen 18 auf, die Überströmungskanäle bilden und die nach dem Verbinden der beiden Zylinderteile 2 und 3 die durch den Trennkolben 10 voneinander getrennten Zylinderräume 11 und 13 miteinander verbinden.

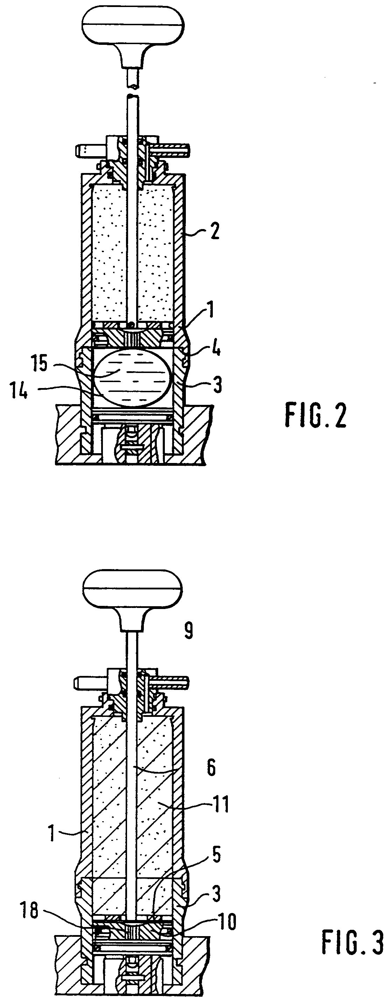

Wie in Fig. 2 gezeigt, wird zu Beginn des Mischvorgangs der obere Zylinderteil 2 mittels der Schnellkupplung 4 mit dem unteren Zylinderteil 3 verbunden. Sodann wird der Mischkolben 5 mittels der Betätigungsstange 6 und des Betätigungsgriffes 9 nach unten in den unteren Zylinderteil 3 hineinverschoben (Fig. 3), wobei der Mischkolben 5 den Trennkolben 10 mitnimmt. Durch den dabei auf den Flüssigkeitsbehälter 14 ausgeübten Druck wird dieser zerstört und das darin enthaltene flüssige Monomer 15 strömt durch die Überströmungskanäle 18 in den darüber befindlichen Zylinderraum 11, in dem sich das pulverförmige Polymer befindet.

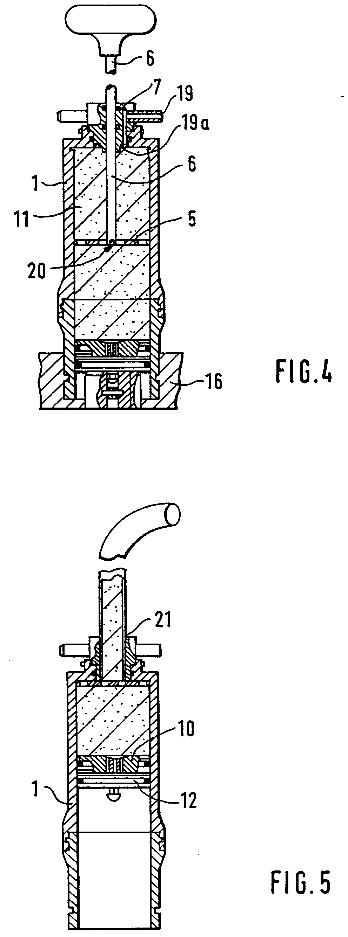

Anschließend wird der Mischkolben 5 mittels der Betätigungsstange 6 auf- und abwärtsbewegt (Fig. 4) und erforderlichenfalls gedreht, wodurch eine innige Durchmischung des pulverförmigen Polymers mit dem flüssigen Monomer erfolgt.

An der Führungsbuchse 7 der Betätigungsstange 6 ist eine Vakuumleitung 19 angeschlossen, die über einen Filter 19a in den Zylinderraum 11 mündet. Durch Anlegen eines Vakuums wird während des Mischvorgangs im Zylinderraum 11 ein Vakuum erzeugt, durch das die Bildung von Poren im gemischten Knochenzement verhindert bzw. wesentlich vermindert wird.

Nach Beendigung des Mischvorgangs wird der Betätigungsstab 6 vom Mischkolben 5 getrennt. Hierzu ist an der Verbindungsstelle zwischen Betätigungsstab 6 und Mischkolben eine Sollbruchstelle 20 vorgesehen, an der der Betätigungsstab 6 durch Ziehen, Drücken oder Drehen vom Mischkolben 5 gelöst werden kann.

Anschließend wird die Führungsbuchse 7 vom Mischzylinder 1 abgenommen und gegen einen Austragsstutzen 21 ausgetauscht (Fig. 5). Der Mischzylinder 1 wird von der Haltevorrichtung 16 abgenommen und mit einem (nicht dargestellten) Austragsantrieb verbunden, beispielsweise einer Handpresse, durch den der Austragskolben 12 in den Mischzylinder 1 hineingedrückt wird. Der Austragskolben 12 nimmt dabei den Trennkolben 10 mit und drückt den im Mischzylinder 1 enthaltenen, gemischten Knochenzement durch den Austragsstutzen 21 heraus.

Abweichend von dem dargestellten Ausführungsbeispiel ist es auch möglich, das flüssige Monomer im unteren Zylinderteil 3 offen bereitzustellen. Beim Verbinden der beiden Zylinderteile 2 und 3 verhindert der das obere Zylinderteil 2 abschließende Trennkolben 10, daß pulverförmiges Polymer aus dem oberen Zylinderteil 2 herausfällt, wenn die beiden Zylinderteile 2 und 3 miteinander verbunden werden. Stattdessen können die beiden Zylinderteile 2 und 3 auch als vorkonfektionierte Kartuschen bereitgestellt werden, die jeweils eines der beiden Ausgangsmaterialien enthalten.

Die beschriebene Vorrichtung kann in vakuumdichter und/oder sterilisierbarer Ausführung ausgelegt werden. Beispielsweise kann die gesamte Vorrichtung nach dem Verbinden der Zylinderteile 2 und 3 sterilisiert werden, bevor sie in den Operationsbereich gebracht wird.

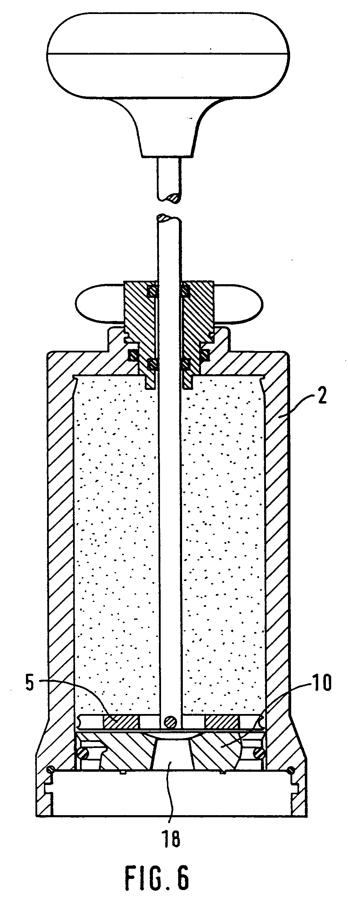

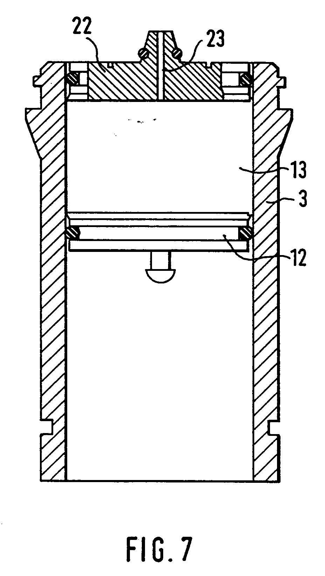

Das in den Fig. 6 und 7 dargestellte Ausführungsbeispiel unterscheidet sich von dem vorher beschriebenen Ausführungsbeispiel im wesentlichen dadurch, daß am oberen Ende des unteren Zylinderteils 3 und somit zwischen dem Austragskolben 12 und dem Trennkolben 10 ein Verschlußkolben 22 angeordnet ist. In dem in den Fig. 6 und 7 gezeigten Zustand, in dem die beiden Zylinderteile 2 und 3 noch voneinander getrennt sind, verschließt der Verschlußkolben 22 den Zylinderraum 13 des unteren Zylinderteils 3. Der axial beweglich im unteren Zylinderteil 3 aufgenommene Verschlußkolben 22 weist ebenfalls eine Bohrung 23 auf, die einen Überströmungskanal bildet.

Wenn die Zylinderteile 2 und 3 entsprechend Fig. 2 miteinander verbunden werden, kommt der Trennkolben 10 zur Anlage am Verschlußkolben 22 und nimmt diesen mit in Richtung auf den Austragskolben 12, wenn der Mischkolben 5 entsprechend der Fig. 3 nach unten gedrückt wird.

Die Verwendung des Verschlußkolbens 22 ermöglicht es, im Zylinderraum 13 des unteren Zylinderteils 3 die ein Ausgangsmaterial für den Knochenzement bildende Flüssigkeit abgeschlossen bereitzustellen, ohne daß hierfür ein gesonderter Flüssigkeitsbehalter 14 verwendet werden müßte.

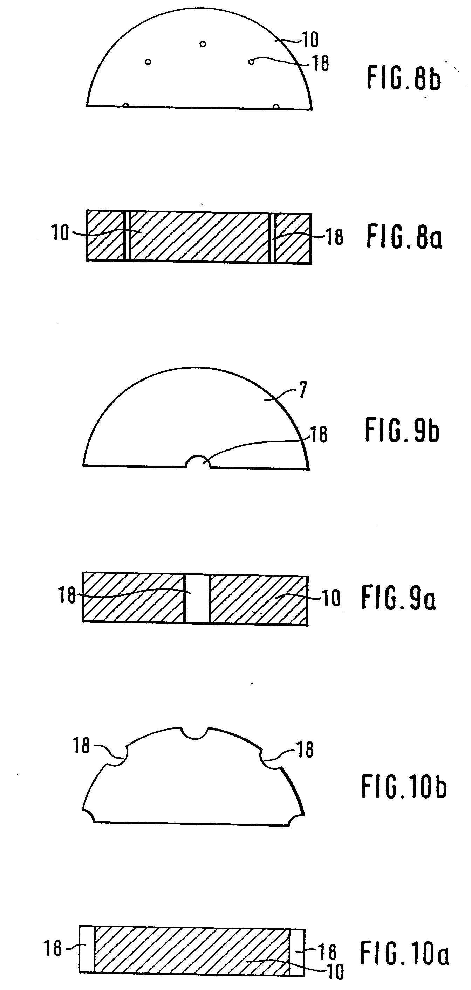

In den Fig. 8-12 sind unterschiedliche Ausführungsbeispiele der Überströmkanäle 18 am Trennkolben 10 schematisch dargestellt.

Beim Beispiel nach Fig. 8 bilden mehrere auf einem Kranz angeordnete Bohrungen die Überströmungskanäle 18. Dadurch dringt das flüssige Monomer in mehreren getrennten Strahlen in das pulverförmige Polymer ein, um eine intensive Durchmischung zu erreichen.

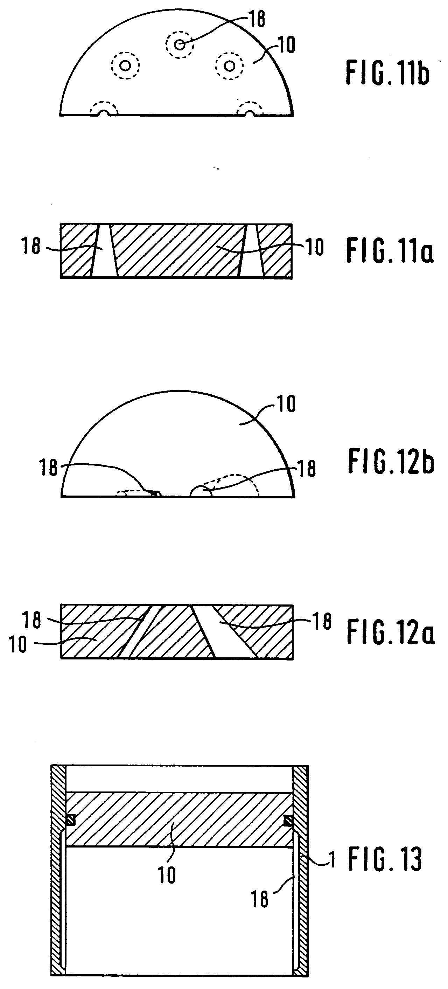

Fig. 9 zeigt schematisch die auch in Fig. 6 dargestellte Ausführungsform, bei der nur eine zentrale Bohrung als Überströmungskanal 18 im Trennkolben 10 vorgesehen ist.

Beim Beispiel nach Fig. 10 sind die Überströmungskanäle 18 von Ausnehmungen am Rand des Trennkolbens 10 gebildet. Dadurch wird ein Überströmen der Flüssigkeit im Randbereich des Mischzylinders ermöglicht.

Fig. 11 zeigt eine Abwandlung gegenüber der Fig. 8. Auch hierbei werden die Überströmungskanäle 18 von mehreren Bohrungen gebildet, die sich zum austragsseitigen Zylinderraum 11 hin verjüngen. Dadurch wird eine Beschleunigung der Flüssigkeitsströmung beim Eintritt in das Pulver erreicht.

Bei der Ausführung nach Fig. 12 sind die die Überströmungskanäle 18 bildenden Bohrungen schräg zur Zylinderachse angeordnet, um eine bessere Durchmischung zu erreichen. Zusätzlich können auch diese Bohrungen verjüngt ausgeführt sein, wie in Fig. 12a) gezeigt ist.

In Fig. 13 ist schematisch angedeutet, daß die Überströmungskanäle 18 durch Längsnuten in der Innenwand des Mischzylinders 1 gebildet werden können.

## Patentansprüche

1. **.** Vorrichtung zum Mischen und Austragen von Knochenzement mit einem zweiteiligen Mischzylinder (1), in dem ein durchbrochener Mischkolben (5) mittels einer an einem Zylinderende abgedichtet herausgeführten Betätigungsstange (6) axial und drehbar bewegbar ist, wobei der Mischzylinder am anderen Zylinderende durch einen lösbar verriegelten Austragskolben (12) verschlossen ist, der axial in den Mischzylinder eindrückbar ist, um den gemischten Knochenzement durch eine gegenüberliegende Austragsöffnung auszutragen, dadurch gekennzeichnet, daß zwischen dem Mischkolben (5) und dem Austragskolben (12) ein Trennkolben (10) axial verschiebbar im Mischzylinder (1) angeordnet ist und daß die durch den Trennkolben (10) voneinander getrennten Zylinderräume (11, 13) des Mischzylinders (1) durch mindestens einen Überströmungskanal (18) miteinander verbunden sind.

2. Vorrichtung nach Anspruch 1, dadurch gekennzeichnet, daß der Überströmungskanal (18) durch mindestens eine Bohrung im Trennkolben (10) gebildet wird.

3. Vorrichtung nach Anspruch 2, dadurch gekennzeichnet, daß der Trennkolben (10) mehrere Bohrungen (18) aufweist.

4. **.** Vorrichtung nach Anspruch 2 oder 3, dadurch gekennzeichnet, daß sich eine oder mehrere Bohrungen (18) zum austragsseitigen Zylinderraum (11) hin verjüngen.

5. Vorrichtung nach einem der Ansprüche 2-4, dadurch gekennzeichnet, daß die Bohrungen (18) schräg zur Zylinderachse verlaufen.

6. Vorrichtung nach Anspruch 1, dadurch gekennzeichnet, daß Ausnehmungen am Rand des Trennkolbens (10) die überströmungskanäle (18) bilden.

7. Vorrichtung nach Anspruch 1, dadurch gekennzeichnet, daß die Überströmungskanäle (18) durch Längsnuten in der Innenwand des Mischzylinders (1) gebildet werden.

8. Vorrichtung nach Anspruch 1, dadurch gekennzeichnet, daß die Betätigungsstange (6) lösbar mit dem Mischkolben (5) verbunden ist und daß eine abnehmbar am Mischzylinder (1) angebrachte Führungsbuchse (7), durch die die Betätigungsstange (6) abgedichtet geführt ist, gegen einen Austragsstutzen (21) austauschbar ist.

9. Vorrichtung nach Anspruch 8, dadurch gekennzeichnet, daß die lösbare Verbindung zwischen der Betätigungsstange (6) und dem Mischkolben (5) durch eine Sollbruchstelle (20) gebildet wird.

10. Vorrichtung nach Anspruch 1, dadurch gekennzeichnet, daß zwischen dem Austragskolben (12) und dem Trennkolben (10) ein axial beweglicher Verschlußkolben (22) angeordnet ist.

11. Vorrichtung nach Anspruch 1, dadurch gekennzeichnet, daß in den beiden Zylinderräumen (11,13) des Mischzylinders (1) vorkonfektionierte Ladungen der Mischungskomponenten aufnehmbar sind.

## Claims

1. Device for mixing and discharging bone cement, with a two-part mixing cylinder (1) in which an openworked mixing plunger (5) can be moved axially and rotatably by means of an actuation rod (6) emerging in a sealed manner at one cylinder end, the mixing cylinder being closed at the other cylinder end by a releasably locked discharging plunger (12) which can be pressed axially into the mixing cylinder in order to discharge the mixed bone cement through an opposite discharge opening, characterized in that a separating plunger (10) is arranged axially displaceably in the mixing cylinder (1) between the mixing plunger (5) and the discharging plunger (12), and in that the cylinder chambers (11, 13) of the mixing cylinder (1) which are separated from each other by the separating plunger (10) are connected to each other via at least one through-flow channel (18).

2. Device according to Claim 1, characterized in that the through-flow channel (18) is formed by at least one bore in the separating plunger (10).

3. Device according to Claim 2, characterized in that the separating plunger (10) has a plurality of bores (18).

4. Device according to Claim 2 or 3, characterized in that one or more bores (18) taper towards the discharge-side cylinder chamber (11).

5. Device according to one of Claims 2 - 4, characterized in that the bores (18) extend inclined with respect to the cylinder axis.

6. Device according to Claim 1, characterized in that recesses at the edge of the separating plunger (10) form the through-flow channels (18).

7. Device according to Claim 1, characterized in that the through-flow channels (18) are formed by longitudinal grooves in the inner wall of the mixing cylinder (1).

8. Device according to Claim 1, characterized in that the actuation rod (6) is connected releasably to the mixing plunger (5), and in that a guide bush (7) which is arranged detachably on the mixing cylinder (1), and through which the actuation rod (6) is guided sealingly, can be replaced by a discharge nozzle (21).

9. Device according to Claim 8, characterized in that the releasable connection between the actuation rod (6) and the mixing plunger (5) is formed by a predetermined break point (20).

10. Device according to Claim 1, characterized in that an axially movable sealing plunger (22) is arranged between the discharging plunger (12) and the separating plunger (10).

11. Device according to Claim 1, characterized in that pre-formulated charges of the components of the mixture can be held in the two cylinder chambers (11, 13) of the mixing cylinder (1).

## Revendications

1. Dispositif pour mélanger et extraire un ciment pour os, comportant un cylindre mélangeur en deux parties (1) dans lequel un piston mélangeur (5), percé, peut être mû axialement et en rotation par une tige de manoeuvre (6) sortant, avec étanchéité, à une extrémité du cylindre, le cylindre mélangeur étant obturé à l'autre extrémité du cylindre par un piston d'extraction (12) qui est verrouillé, avec liberté de déverrouillage, et qui peut s'enfoncer dans le cylindre mélangeur pour extraire le ciment pour os, mélangé, par une ouverture d'extraction située en face, caractérisé par le fait qu'entre le piston mélangeur (5) et le piston d'extraction (12) un piston séparateur (10) est disposé avec liberté de coulisser axialement dans le cylindre mélangeur (1) et que les espaces (11, 13) du cylindre mélangeur (1) séparés l'un de l'autre par le piston séparateur (10) sont reliés l'un à l'autre par au moins un canal de débordement (18).

2. Dispositif selon la revendication 1, caractérisé par le fait que le canal de débordement (18) est formé par au moins un perçage dans le piston séparateur (10).

3. Dispositif selon la revendication 2, caractérisé par le fait que le piston séparateur (10) présente plusieurs perçages (18).

4. Dispositif selon la revendication 2 ou 3, caractérisé par le fait qu'un ou plusieurs perçages (18) vont en se rétrécissant en direction de l'espace du cylindre (11) situé du côté de l'extraction.

5. Dispositif selon l'une des revendications 2-4, caractérisé par le fait que les perçages (18) sont orientés perpendiculairement à l'axe du cylindre.

6. Dispositif selon la revendication 1, caractérisé par le fait que des évidements sur le bord du piston séparateur (10) forment les canaux de débordement (18).

7. Dispositif selon la revendication 1, caractérisé par le fait queque les canaux de débordement (8) sont formés par des rainures longitudinales dans la paroi intérieure du cylindre mélangeur (1).

8. Dispositif selon la revendication 1, caractérisé par le fait que la tige de manoeuvre (6) est reliée, de façon amovible, avec le piston mélangeur (5) et qu'un manchon de guidage (7) qui est rapporté, de façon amovible, sur le cylindre mélangeur (1) et par lequel passe, avec étanchéité, la pièce de manoeuvre (6), peut être échangé contre une tubulure d'extraction (21).

9. Dispositif selon la revendication 8, caractérisé par le fait que la liaison amovible entre la tige de manoeuvre (6) et le piston mélangeur (5) est formée par une position de rupture préférentielle (20).

10. Dispositif selon la revendication 1, caractérisé par le fait qu'entre le piston d'extraction (12) et le piston séparateur (10) est disposé un piston d'obturation (22) axialement mobile.

11. Dispositif selon la revendication 1, caractérisé par le fait que dans les deux espaces (11, 13) du cylindre mélangeur (1) peuvent être reçues des charges préconfectionnées des composants du mélange.
